# EUROPÄISCHE PATENTANMELDUNG

(11) **EP 2 031 530 A2**
(43) Veröffentlichungstag der Anmeldung: **04.03.2009**
(21) Anmeldenummer: 08104955.3
(22) Anmeldetag: 04.08.2008
(51) Int. Cl.: G06F 19/00

(54) **Verfahren zur rechnergestützten Analyse eines Objekts**

(30) Priorität: 09.08.2007 DE 102007037633; 16.11.2007 DE 102007054835
(71) Anmelder: Siemens Aktiengesellschaft, 80333 München (DE)
(72) Erfinder: Berger, Michael, 82110 Germering (DE); Beyer, Dagmar, 81371 München (DE); Hubauer, Thomas, 81475 München (DE)

(57) **Zusammenfassung**

Die Erfindung betrifft ein Verfahren zur rechnergestützten Analyse eines Objekts. Unter Objekt sind hierbei beliebige Gegenstände bzw. Lebewesen zu verstehen, und das Verfahren kann in beliebigen technischen Gebieten eingesetzt werden, beispielsweise in den Bereichen Medizin, Sicherheitstechnik, Verkehrstechnik und dergleichen. Mit dem erfindungsgemäßen Verfahren werden mit Sensormitteln die Informationen zu dem Objekt für eine Mehrzahl von zeitlich aufeinander folgenden Zeitpunkten bzw. Zeiträumen erfasst. Anschließend wird basierend auf den einzelnen Zeitpunkten bzw. Zeiträumen eine Zustands- bzw. Situationsbeschreibung des Objekts mit einem ersten Analyseverfahren ermittelt, wobei für dieses Verfahren vorzugsweise auf einer Ontologie basierende Schlussfolgerungs-Verfahren unter Verwendung von Reasonern eingesetzt werden. Die Zustandsbeschreibungen des Objekts bilden dann wiederum Eingangsgrößen für ein zweites Analyseverfahren, mit dem eine zeitliche Verhaltensbeschreibung des Objekts ermittelt wird. Dieses zweite Analyseverfahren verwendet vorzugsweise ebenfalls auf einer Ontologie basierende Schlussfolgerungs-Verfahren. Durch die Kombination von zwei Analyseverfahren können erfindungsgemäß auf einfache Weise einzelne Situationen zu verschiedenen Zeitpunkten erkannt werden und hieraus entsprechende zeitliche Verhaltensbeschreibungen abgeleitet werden. Ein Anwendungsbereich ist beispielsweise ein medizinisches Überwachungssystem, bei dem medizinische Notfälle bzw. komplexe Krankheitsbilder durch Ermittlung der zeitlichen Verhaltensbeschreibung erfasst werden. Ebenso kann das erfindungsgemäße Verfahren z.B. in Sicherheitssystemen eingesetzt werden, um verdächtige Verhaltensweisen von Personen zu erkennen.

## Beschreibung

Die Erfindung betrifft ein Verfahren zur rechnergestützten Analyse eines Objekts sowie ein entsprechendes Computerprogrammprodukt.

Verhaltenserkennung spielt in einer Vielzahl von technischen Bereichen eine wesentliche Rolle. Auf dem Gebiet der Medizin kann über Verhaltenserkennung der Zustand eines Patienten ermittelt werden und es können akute Notfälle erkannt werden. In der Sicherheitstechnik kann aus dem Verhalten einer Person beispielsweise auf einen Einbruchsversuch geschlossen werden. In der Verkehrstechnik spielt die Erkennung des Verhaltens des Fahrers und des Fahrzeugs eine wichtige Rolle bei Fahrerassistenzsystemen.

Für Verfahren zur rechnergestützten Verhaltenserkennung ist es wesentlich, dass nicht nur zu einem einzelnen Zeitpunkt ein "Schnappschuss" des entsprechenden Systems betrachtet wird. Vielmehr muss eine zeitliche Folge von Zuständen bzw. Situationen, in denen sich das System befindet, betrachtet werden. Je nach Zielsetzung kann die Zeitgranulität, d.h. der sinnvolle Abstand zwischen einzelnen Situationen, stark variieren. Bei einem Sturz einer überwachten Person charakterisiert gerade die plötzliche Situationsänderung zwischen dem Zustand "stehend" und "liegend" die Notfallsituation, wohingegen der Zustand "stehend" bzw. "liegend" für sich allein betrachtet keine Aussage zulässt. Im Unterschied dazu verschlechtert sich in vielen technischen Systemen der Zustand des Systems oftmals nicht rapide, sondern schleichend. Eine Situationsänderung kann dabei nur als alarmierend und interventionswürdig erkannt werden, wenn sie über längere Zeit betrachtet wird.

Aus dem Stand der Technik sind verschiedene rechnergestützte Verfahren zur Situationserkennung bekannt. Eine erste Klasse solcher Verfahren beschränkt sich darauf, zu einem vorgegebenen Zeitpunkt das System zu betrachten und die Situation zu klassifizieren. Die fehlende Beobachtung der zeitlichen Änderung der Situation ermöglicht es dabei nicht, ein entsprechendes Verhalten abzuleiten.

Eine weitere Klasse von rechnergestützten Verfahren ermöglicht die Verhaltenserkennung unter Berücksichtigung des zeitlichen Verlaufs von beobachteten Ereignissen. Die Druckschrift [1] zeigt beispielsweise ein Überwachungssystem für Schwimmbecken, um automatisch das Ertrinken von Personen zu detektieren. In der genannten Druckschrift werden stark spezialisierte Algorithmen zur Verhaltensdetektion verwendet, ohne dass explizit Situationsbeschreibungen des Objekts zu unterschiedlichen Zeitpunkten bzw. Zeiträumen betrachtet werden.

Aufgabe der Erfindung ist es, ein Verfahren zur rechnergestützten Analyse eines Objekts zu schaffen, welches einfach und effizient das zeitliche Verhalten des Objekts erkennt.

Diese Aufgabe wird durch die unabhängigen Patentansprüche gelöst. Weiterbildungen der Erfindung sind in den abhängigen Ansprüchen definiert.

In dem erfindungsgemäßen Verfahren werden für eine Mehrzahl von zeitlich aufeinander folgenden Zeitpunkten und/oder Zeiträumen jeweils Informationen zu dem Objekt mit einem oder mehreren Sensormitteln erfasst. Der Begriff Objekt ist hierbei weit auszulegen und kann sowohl Gegenstände als auch Personen betreffen. Ein Objekt kann z.B. ein zu überwachendes Lebewesen umfassen, insbesondere einen Menschen, dessen Verhalten z.B. in einem medizinischen Überwachungssystem analysiert wird. Ebenso kann ein Objekt einen räumlichen Bereich mit darin enthaltenen Gegenständen und/oder Personen umfassen, beispielsweise einen Raum oder ein Zimmer. Das Objekt kann auch beliebige technische Systeme oder Teile davon betreffen. Beispielsweise kann das Objekt eine Automatisierungsanlage und/oder ein Teil einer Automatisierungsanlage und/oder ein Transportsystem und/oder ein Teil eines Transportsystems, beispielsweise ein Schienenfahrzeug, umfassen. Das Objekt kann ferner ein Fahrzeug und den Fahrer eines Fahrzeugs umfassen, deren Verhalten in einem Fahrerassistenzsystem analysiert wird. Der Begriff "Objekt" ist auch dahingehend zu verstehen, dass ein Objekt viele unterschiedliche Komponenten umfassen kann und nicht auf einen einzelnen Gegenstand bzw. eine einzelne Person beschränkt ist.

In dem erfindungsgemäßen Verfahren werden für einen jeweiligen Zeitpunkt und/oder Zeitraum der Mehrzahl von aufeinander folgenden Zeitpunkten bzw. Zeiträumen ein erstes Analyseverfahren auf die für den jeweiligen Zeitpunkt und/oder Zeitraum erfassten Informationen angewendet, wodurch Zustandsbeschreibungen des Objekts zu den jeweiligen Zeitpunkten und/oder Zeiträumen erhalten werden. Auf mehrere Zustandsbeschreibungen des Objekts wird dann ein weiteres, zweites Analyseverfahren angewendet, wodurch eine zeitliche Verhaltensbeschreibung des Objekts erhalten wird. Dieses zweite Analyseverfahren kann auch wiederholt auf neu erfasste Zustandsbeschreibungen angewendet werden, um Veränderungen im Verhalten zu erfassen. Die Zustandsbeschreibungen zu den verschiedenen Zeitpunkten stellen somit wiederum Eingaben für das zweite Analyseverfahren dar, d.h. sie werden wie mit Sensormitteln erfasste Informationen aufgefasst. Durch die erfindungsgemäße Verknüpfung eines ersten Analyseverfahrens zur Erkennung des Zustands des Objekts in der Form von Zustands- bzw. Situationsbeschreibungen mit einem zweiten Analyseverfahren zur Ermittlung eines entsprechenden Verhaltens des Objekts aus den zeitlich aufeinander folgenden Situationen wird eine einfache und generische Möglichkeit geschaffen, das Verhalten von beliebigen Objekten zu analysieren und daraus eine entsprechende Verhaltensbeschreibung abzuleiten.

In einer besonders bevorzugten Ausführungsform wird als erstes bzw. zweites Analyseverfahren ein auf einer Ontologie basierendes Schlussfolgerungs-Verfahren eingesetzt. Durch die Ontologie wird das Wissen über das Objekt durch Begriffe und Relationen festgelegt, und mit Hilfe des Schlussfolgerungs-Verfahrens werden dann aus der Ontologie die entsprechenden Zustands- bzw. Verhaltensbeschreibungen abgeleitet. Auf Ontologie basierende Schlussfolgerungs-Verfahren sind auf dem Gebiet der Informatik hinlänglich bekannt. In einer bevorzugten Ausführungsform werden dabei Schlussfolgerungs-Verfahren verwendet, welche auf einer deterministischen oder probabilistischen Beschreibungslogik beruhen, wobei die Zustandsbeschreibung oder Verhaltensbeschreibung des Objekts mit Hilfe eines oder mehrerer Reasoner ermittelt wird. Bei der Beschreibungslogik handelt es sich um eine entscheidbare Untermenge der Prädikatenlogik, und in einer bevorzugten Ausführungsform wird die Beschreibungslogik durch die hinlänglich aus dem Stand der Technik bekannte Beschreibungssprache OWL-DL (OWL-DL = Web Ontology Language Description Language) repräsentiert. Die Implementierung von Schlussfolgerungs-Verfahren in der Form von Reasonern ist aus dem Stand der Technik bekannt, und erfindungsgemäß werden im Zusammenhang mit Beschreibungslogiken beispielsweise die Reasoner Pellet und/oder Racer und/oder FaCT++ eingesetzt.

In einer weiteren, besonders bevorzugten Ausführungsform des erfindungsgemäßen Verfahrens werden zur Durchführung des ersten Analyseverfahrens eine Mehrzahl von Reasonern verwendet, wobei die Reasoner auf Informationen zu dem Objekt angewendet werden, welche zu unterschiedlichen, insbesondere aufeinander folgenden, Zeitpunkten und/oder Zeiträumen erfasst wurden. Durch diese parallele Verwendung von Reasonern kann die Rechenzeit des erfindungsgemäßen Verfahrens verkürzt werden, da gleichzeitig zu mehreren Zeitpunkten mit mehreren Reasonern Zustandsbeschreibungen des Objekts ermittelt werden können.

In einer bevorzugten Ausführungsform der Erfindung wird eine besonders einfache Verknüpfung einer Zustandsbeschreibung des Objekts zu einem jeweiligen Zeitpunkt und/oder Zeitraum mit der Information über den Zeitpunkt und/oder Zeitraum dadurch erreicht, dass der jeweiligen Zustandsbeschreibung ein entsprechender Zeitstempel zugeordnet wird.

Anstatt bzw. zusätzlich zu der oben beschriebenen Verwendung von Schlussfolgerungs-Verfahren, beispielsweise basierend auf Reasonern, kann der erste oder zweite Analyseschritt gegebenenfalls auch auf andere Weise eine entsprechende Zustands- bzw. Verhaltensbeschreibung des Objekts ableiten. Insbesondere können hinlänglich aus dem Stand der Technik bekannte kausale Netze hierzu eingesetzt werden, z.B. Bayes-Netze.

In einer besonders bevorzugten Ausführungsform des erfindungsgemäßen Verfahrens wird die mit dem zweiten Analyseverfahren ermittelte Verhaltensbeschreibung mit vorbestimmten Verhaltensbeschreibungen verglichen, und bei Abweichung von den vorbestimmten Verhaltensbeschreibungen wird eine Meldung, insbesondere ein Alarmsignal, ausgegeben. Auf diese Weise kann das erfindungsgemäße Verfahren sehr gut in entsprechenden Überwachungssystemen eingesetzt werden.

Die Sensormittel, mit denen Informationen zu dem Objekt erfasst werden, können beliebig ausgestaltet sein. Es kann sich hierbei beispielsweise um eine Kamera und/oder einen Bewegungssensor und/oder einen Geräuschsensor und/oder Sensoren zur Erfassung von Vitalwerten eines Lebewesens handeln.

Neben dem oben beschriebenen Verfahren betrifft die Erfindung ferner ein Computerprogrammprodukt mit einem auf einem maschinenlesbaren Träger gespeicherten Programmcode zur Durchführung des erfindungsgemäßen Verfahrens, wenn das Programm auf einem Rechner abläuft.

Ausführungsbeispiele der Erfindung werden nachfolgend anhand der beigefügten Figur detailliert beschrieben.

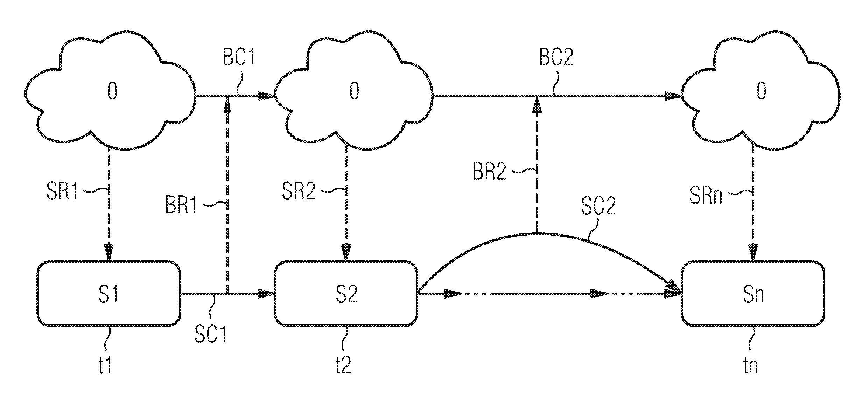

- Fig. 1: zeigt eine schematische Darstellung, welche die in einer Ausführungsform des erfindungsgemäßen Verfahrens durchgeführten Schritte verdeutlicht.

Gemäß der nachfolgend beschriebenen Ausführungsform des erfindungsgemäßen Verfahrens wird zu einzelnen, aufeinander folgenden Zeitpunkten t1, t2, ..., tn jeweils eine Situation zu diesen Zeitpunkten betreffend ein zu analysierendes Objekt O erkannt. Die Situationserkennung ist in Fig. 1 beispielhaft für die Zeitschritte t1, t2 bzw. tn mit SR1, SR2 bzw. SRn bezeichnet. Die entsprechend erkannte Situation ist für die Zeitschritte t1, t2 und tn mit S1, S2 und Sn wiedergegeben. Die Erkennung der einzelnen Situationen erfolgt hierbei mit Hilfe von entsprechenden Sensormitteln, welche das zu analysierende Objekt erfassen. Das Objekt kann hierbei ein beliebiges beobachtbares Szenario betreffen. Die Erfindung könnte beispielsweise in einem medizinischen Überwachungssystem eingesetzt werden, bei dem das beobachtete Objekt eine menschliche Person darstellt. Von dieser Person werden zu den einzelnen Zeitpunkten t1, t2, ..., tn Situationen in der Form einer Momentaufnahme der Vitalwerte der Person, d.h. beispielsweise Herzschlag, Blutdruck, Atemfunktion und dergleichen erfasst. Darüber hinaus besteht die Möglichkeit, anstatt zu festen Zeitpunkten die Verhaltensweisen der Person innerhalb vorgegebener, aufeinander folgender Zeiträume zu erfassen, um beispielsweise komplexere Krankheitsbilder, wie etwa eine Depression, zu erkennen und zu analysieren. In diesem Fall entspricht eine Situation einer Beobachtung über einen Zeitraum, d.h. einem "Verhaltensprofil" der überwachten Person in dem Zeitraum. Dieses Profil kann beispielsweise aus üblichen Schlafenszeiten, Aktivitäten, Nutzungsmustern der Räumlichkeiten etc. bestehen.

Ein anderes Anwendungsbeispiel der Erfindung ist die Erfassung von vorbestimmten Größen in Räumen, beispielsweise in einem Besprechungsraum. In einem solchen Besprechungsraum können die Sensormittel beispielsweise Sensoren betreffen, welche erfassen, ob in dem Raum Licht angeschaltet ist oder nicht. Ebenso können die Sensoren Geräuschsensoren betreffen, mit denen ermittelt werden kann, ob in dem Raum gesprochen wird. Weitere Sensoren können beispielsweise in den Stühlen des Besprechungsraums integriert sein, um zu ermitteln, ob und wie viele Personen in dem Besprechungsraum anwesend sind. Ebenso könnte ermittelt werden, ob eine Person an dem Pult im Besprechungsraum steht, beispielsweise mit einer entsprechenden Kamera, deren Bild analysiert wird. Aus all diesen Größen kann dann eine entsprechende Situation zu einem bestimmten Zeitpunkt erkannt werden, beispielsweise ob der Raum frei ist, ob ein Vortrag gehalten wird oder ob eine Diskussion stattfindet.

Ein weiteres Beispiel einer Situationserkennung betrifft die Detektion abgestellter Gepäckstücke, beispielsweise in dem Warteraum eines Zugbahnhofs. Hierbei kann beispielsweise mittels einer Videokamera das Geschehen in dem Warteraum verfolgt werden und mittels einer Bildanalyse des aufgenommenen Videomaterials können Personen und Gegenstände sowie die räumlichen Beziehungen dazwischen als Situation erkannt werden. Ein Beispiel einer solchen erkannten Situation ist die Zustandsbeschreibung "Person P1 trägt Gegenstand O2". Die Gegenstände und deren Beziehungen entsprechen somit den einzelnen Situationen S1, S2, ..., Sn gemäß Fig. 1.

Wie sich aus den obigen Ausführungen ergibt, kann mit dem erfindungsgemäßen Verfahren auf beliebige Weise eine Situation zu aufeinander folgenden Zeitpunkten bzw. Zeiträumen von beliebigen Objekten erkannt werden. In einer bevorzugten Ausführungsform der Erfindung werden zur Situationserkennung rechnergestützte Analyseverfahren basierend auf sog. Reasonern eingesetzt. Reasoner stellen Verfahren zum Ableiten von Schlussfolgerungen basierend auf einer Repräsentation von Wissen dar, wodurch neues Wissen ermittelt werden kann. Reasoner verwenden hierzu üblicherweise eine Beschreibungslogik, bei der es sich um eine Untermenge der dem Fachmann bekannten Prädikatenlogik handelt. Die Beschreibungslogik zeichnet sich dadurch aus, dass sie entscheidbar ist. Beschreibungslogiken unterteilen das dargestellte Wissen üblicherweise in eine sog. T-Box, welche sämtliche Konzepte des dargestellten Modells beschreibt, sowie eine A-Box, welche alle Instanzen der Konzepte, sowie ihre Beziehungen zueinander enthält. Ein Konzept beschreibt hierbei eine Klasse gleichartiger Objekte, wohingegen eine Instanz ein konkretes Objekt darstellt. Im Zusammenhang mit dem oben beschriebenen System zur Detektion abgestellter Gepäckstücke könnte ein Konzept der Oberbegriff "Koffer" sein, wohingegen eine Instanz des Konzepts für einen einzelnen, eindeutig bestimmbaren Koffer steht.

Beschreibungslogiken werden oft zur Darstellung einer Ontologie benutzt, welche die Wissensrepräsentation eines formal definierten Systems von Begriffen und Relationen darstellt. Beschreibungslogiken sowie Ontologien sind hinlänglich aus dem Stand der Technik bekannt, und werden deshalb hier nicht weiter im Detail erläutert. In einer bevorzugten Ausführungsform wird zur Beschreibung der dem betrachteten Objekt zu Grunde liegenden Ontologie die Ontologie-Sprache OWL-DL eingesetzt. Basierend auf dieser Sprache zur Wissensrepräsentation werden dann mit einem entsprechenden Reasoner Schlussfolgerungen abgeleitet. Reasoner sind ebenfalls hinlänglich aus dem Stand der Technik bekannt und werden deshalb nicht im Detail erläutert. Gemäß der hier beschriebenen Ausführungsform der Erfindung können als Reasoner beispielsweise Racer, Pellet bzw. FaCT++ eingesetzt werden.

Wie sich aus den obigen Ausführungsformen ergibt, werden erfindungsgemäß zunächst in jeweiligen Zeitschritten t1, t2, ..., tn separat entsprechende Situationen S1, S2, ..., Sn, z.B. basierend auf Beschreibungslogiken mit Hilfe von Reasonern, erkannt. Den einzelnen Situationen sind hierbei Zeitinformationen zugeordnet, welche explizit durch Zeitstempel, die Nutzung temporaler Logiken und dergleichen gegeben sein können, welche jedoch auch implizit in den einzelnen erkannten Situationen, beispielsweise durch eine Nachfolger-Relation, gegeben sein können.

Wie aus Fig. 1 ersichtlich ist, treten zwischen den einzelnen Zeitschritten t1, t2 usw. Situationsänderungen auf, wobei in Fig. 1 die Situationsänderung SC1 zwischen dem Zeitschritt t1 und t2 wiedergegeben ist sowie eine Situationsänderung SC2, welche eine Mehrzahl von Zeitschritten zwischen t2 und tn umfasst. Die Situationsänderungen haben hierbei ihre Ursache in einer entsprechenden Verhaltensänderung des beobachteten Objekts, wobei die der Situationsänderung SC1 zugeordnete Verhaltensänderung mit BC1 und die der Situation SC2 zugeordnete Verhaltensänderung mit BC2 bezeichnet ist. Ein wesentlicher Aspekt des erfindungsgemäßen Verfahrens besteht nunmehr darin, basierend auf den sich verändernden Situationen die entsprechende Verhaltensänderung zu erkennen und eine zeitliche Verhaltensbeschreibung abzuleiten. Dies erfolgt mit einem weiteren Analyseverfahren, wobei dieses Analyseverfahren nunmehr nicht mehr unmittelbar auf mit Sensormitteln erfassten Größen angewendet wird. Vielmehr stellen die einzelnen erkannten Situationen S1, S2, ..., Sn die Eingangsgrößen des weiteren Analyseverfahrens dar. Dieses weitere Analyseverfahren ist in Fig. 1 mit BR1 bzw. BR2 bezeichnet, wobei das Verfahren BR1 eine Verhaltensbeschreibung aus der Situationsänderung SC1 und das Verfahren BR2 eine Verhaltensbeschreibung aus der Situationsänderung SC2 ableitet. Analog zu dem oben beschriebenen Verfahren zur Situationserkennung kann hierbei wiederum ein auf einer Beschreibungslogik basierender Reasoner verwendet werden, der als Eingangsgrößen nunmehr Situationen zu aufeinander folgenden Zeitpunkten erhält und hieraus basierend auf der Beschreibungslogik neues Wissen ableitet, welches durch eine Verhaltensbeschreibung des Objekts repräsentiert wird.

In der hier beschriebenen Ausführungsform der Erfindung werden somit Reasoner zur Situationserkennung in den einzelnen Zeitschritten t1, t2, ..., tn eingesetzt und ein weiterer Reasoner wird dann zum Ableiten einer Verhaltensbeschreibung aus den zeitlich aufeinander folgenden Situationen verwendet. Um mit einem solchen Verfahren möglichst viele, nur kurze Zeitabstände zueinander versetzte Situationen zu erfassen, werden in einer bevorzugten Ausführungsform mehrere Reasoner parallel zur Situationserkennung für mehrere Zeitpunkte genutzt. Somit können in engen Zeitabständen Situationen erkannt werden, da nicht darauf gewartet werden muss, bis der Reasoner die Situationserkennung abgeschlossen hat und eine Situationserkennung für einen neuen Zeitpunkt durchführen kann. Vielmehr kann bereits eine neue Situation mit einem parallel genutzten Reasoner analysiert werden, während der zu einem vorhergehenden Zeitpunkt verwendete Reasoner noch mit der Analyse der Situation beschäftigt ist.

Wie bereits oben dargelegt wurde, können mit dem erfindungsgemäßen Verfahren beliebige Objekte analysiert werden. In dem oben beschriebenen medizinischen Überwachungssystem können aus den einzelnen erkannten Situationen in der Form von Verhaltensprofilen Abweichungen von bisherigen Verhaltensmustern erkannt werden und hieraus z.B. frühzeitig Symptome einer Depression erkannt werden. Das depressive Verhalten kann sich hierbei aus entsprechenden Abweichungen des Verhaltens der Person im Hinblick auf Schlafzeiten und Aktivitätszeiten ergeben. In dem obigen Beispiel betreffend die Erfassung von Situationen in einem Besprechungsraum könnte beispielsweise ermittelt werden, wann eine Besprechung in dem Raum beginnt bzw. zu Ende ist.

In dem oben beschriebenen Beispiel betreffend die Detektion abgestellter Gepäckstücke können aus den einzelnen Situationen betreffend ein Gepäckstück und eine Person, die das Gepäckstück abgestellt hat, verdächtigte Verhaltensweisen abgeleitet werden. Beispielsweise kann mit Hilfe eines passend erstellten Wissensmodells (Ontologie) die verdächtigte Verhaltensweise erkannt werden, dass eine Person einen Koffer abgestellt hat und sich anschließend von dem Gegenstand entfernt hat. Diese Verhaltensweise deutet insbesondere darauf hin, dass sich in dem Koffer ein gefährlicher Gegenstand, beispielsweise explosives Material, befindet. Das erfindungsgemäße Verfahren könnte somit in einem Überwachungssystem in einem Bahnhof eingesetzt werden, wobei bei Auftreten einer verdächtigen Verhaltensweise eine Warnmeldung an eine Leitstelle ausgegeben wird, woraufhin entsprechende Sicherheitsmaßnahmen, beispielsweise die Evakuierung des Bahnhofs, eingeleitet werden können.

Ein weiterer Anwendungsbereich der Erfindung ist die Überwachung eines Automatisierungssystems bzw. eines Teils eines Automatisierungssystems, wobei der Zustand des Automatisierungssystems wiederum mit entsprechenden Sensormitteln zu vorgegebenen Zeitpunkten erfasst wird und hieraus eine Verhaltensbeschreibung des Automatisierungssystems abgeleitet wird, um beispielsweise verdächtige Vorgänge in der Automatisierungsanlage zu erkennen und das temporäre Abschalten der Anlage zur Vermeidung von weitergehenden Schäden zu initiieren.

Die oben beschriebene Ausführungsform des erfindungsgemäßen Verfahrens weist eine Reihe von Vorteilen auf. Insbesondere wird durch das Verfahren ein generisches und durch Austausch der zu Grunde liegenden Ontologie konfigurierbares System zur zeitbasierten Erkennung von Verhalten geschaffen. Durch explizite Betrachtung der zeitlichen Komponente können genauere Aussagen getroffen werden, als es durch das reine Betrachten von "Schnappschüssen" zu vorgegebenen Zeitpunkten möglich wäre. Die Nutzung von Beschreibungslogik-Reasonern zur Situations- bzw. Verhaltenserkennung erlaubt dabei einen Kompromiss zwischen Ausdrucksstärke und Entscheidbarkeit sowie die Nutzung erprobter und effizienter Bausteine. Neben der Verwendung von deterministischen Beschreibungslogiken, welche eine eindeutige Schlussfolgerung ausgeben, können in der Erfindung gegebenenfalls auch probabilistische Beschreibungslogiken eingesetzt werden, welche Situationen und Verhaltensweisen nach ihrer "Zutreffwahrscheinlichkeit" priorisieren und somit insbesondere im Bereich der Entscheidungsunterstützung einen deutlichen Mehrwert liefern.

Durch die oben beschriebene Verteilung der Situationserkennung auf mehrere, parallel genutzte Reasoner ergeben sich weitere Vorteile. Dauert beispielsweise das Erkennen einer einzelnen Situation fünf Sekunden, so kann ein einzelner Reasoner auch nur Zeitschritte zwischen aufeinander folgenden Zeitpunkten mit mindestens fünf Sekunden Abstand betrachten. Durch Parallelschaltung von beispielsweise fünf Reasonern kann die minimale Dauer eines Zeitschritts auf eine Sekunde verringert werden. Durch die Verteilung auf mehrere Reasoner können auch verschiedene Reasoning-Techniken für die Erkennung von Situationen sowie die Verhaltenserkennung genutzt werden. Beispielsweise könnten Situationen in den einzelnen Zeitschritten mittels eines deterministischen Verfahrens erkannt werden, wohingegen die anschließende Verhaltenserkennung basierend auf den erkannten Situationen mit probabilistischen Verfahren stattfindet, bei denen Gewichtungen mittels Wahrscheinlichkeiten vorgenommen werden.

### Literaturverzeichnis

[1] How-Lung Eng, Kar-Ann Toh, Alvin H. Kam, Junyian Wang und Wei-Yun Yau, "An automatic drowning detection surveillance system for challenging outdoor pool environments", Proc. of the Ninth IEEE International Conference on Computer Vision (ICCV 2003).

## Patentansprüche

1. Verfahren zur rechnergestützten Analyse eines Objekts (O), bei dem
- für eine Mehrzahl von zeitlich aufeinander folgenden Zeitpunkten (t1,..., tn) und/oder Zeiträumen jeweils Informationen zu dem Objekt (O) mit einem oder mehreren Sensormitteln erfasst werden;
- für einen jeweiligen Zeitpunkt (t1,..., tn) und/oder Zeitraum ein erstes Analyseverfahren (SR1, SR2) auf die für den jeweiligen Zeitpunkt (t1,..., tn) und/oder Zeitraum erfassten Informationen angewendet wird, wodurch Zustandsbeschreibungen des Objekts (O) zu den jeweiligen Zeitpunkten (t1,..., tn) und/oder Zeiträumen erhalten werden;
- auf mehrere Zustandsbeschreibungen des Objekts (O) ein zweites Analyseverfahren (BR1, BR2) angewendet wird, wodurch eine zeitliche Verhaltensbeschreibung des Objekts (O) erhalten wird.

2. Verfahren nach Anspruch 1, bei dem das erste und/oder zweite Analyseverfahren (SR1, SR2; BR1, BR2) ein auf einer Ontologie basierendes Schlussfolgerungs-Verfahren umfasst.

3. Verfahren nach Anspruch 2, bei dem das Schussfolgerungs-Verfahren auf einer deterministischen oder probabilistischen Beschreibungslogik beruht und die Zustandbeschreibung und/oder Verhaltensbeschreibung des Objekts (O) mit Hilfe eines oder mehrerer Reasoner ermittelt wird.

4. Verfahren nach Anspruch 3, bei dem die Beschreibungslogik durch OWL-DL repräsentiert wird.

5. Verfahren nach Anspruch 3 oder 4, bei dem der eine oder die mehreren Reasoner Pellet und/oder Racer und/oder FaCT++ umfassen.

6. Verfahren nach einem der Ansprüche 3 bis 5, bei dem zur Durchführung des ersten Analyseverfahrens (SR1, SR2) eine Mehrzahl von Reasonern verwendet wird, wobei die Reasoner auf Informationen zu dem Objekt (O) angewendet werden, welche zu unterschiedlichen, insbesondere aufeinander folgenden, Zeitpunkten (t1,...,tn) und/oder Zeiträumen erfasst wurden.

7. Verfahren nach einem der vorhergehenden Ansprüche, bei dem einer Zustandsbeschreibung des Objekts (O) zu einem jeweiligen Zeitpunkt (t1,..., tn) und/oder Zeitraum die Information über den Zeitpunkt (t1,..., tn) und/oder Zeitraum durch einen Zeitstempel zugeordnet wird.

8. Verfahren nach einem der vorhergehenden Ansprüche, bei dem das erste und/oder zweite Analyseverfahren (SR1, SR2; BR1, BR2) auf kausalen Netzen beruhen, insbesondere auf einem Bayes-Netz.

9. Verfahren nach einem der vorhergehenden Ansprüche, wobei die mit dem zweiten Analyseverfahren ermittelte Verhaltensbeschreibung mit vorbestimmten Verhaltensbeschreibungen verglichen wird, und bei Abweichung von den vorbestimmten Verhaltensbeschreibungen eine Meldung, insbesondere ein Alarmsignal, ausgegeben wird.

10. Verfahren nach einem der vorhergehenden Ansprüche, bei dem das zu analysierende Objekt (O) ein Lebewesen, insbesondere einen Menschen, umfasst, wobei das Verfahren vorzugsweise in einem medizinischen Überwachungssystem eingesetzt wird.

11. Verfahren nach einem der vorhergehenden Ansprüche, bei dem das zu analysierende Objekt (O) einen räumlichen Bereich mit darin enthaltenen Gegenständen und/oder Personen umfasst, insbesondere einen Raum und/oder ein Zimmer.

12. Verfahren nach einem der vorhergehenden Ansprüche, bei dem das zu analysierende Objekt (O) ein technisches System oder ein Teil eines technischen Systems umfasst.

13. Verfahren nach Anspruch 12, bei dem das technische System eine Automatisierungsanlage und/oder einen Teil einer Automatisierungsanlage und/oder ein Transportsystem und/oder ein Teil eines Transportsystems umfasst.

14. Verfahren nach einem der vorhergehenden Ansprüche, bei dem das Verfahren in einem Fahrerassistenzsystem eingesetzt wird, wobei das Verhalten des Fahrers eines Fahrzeugs sowie das Verhalten des Fahrzeugs analysiert werden.

15. Verfahren nach einem der vorhergehenden Ansprüche, bei dem das oder die Sensormittel eine Kamera und/oder einen Bewegungssensor und/oder einen Geräuschsensor und/oder Sensoren zur Erfassung von Vitalwerten eines Lebewesens umfassen.

16. Computerprogrammprodukt mit einem auf einem maschinenlesbaren Träger gespeicherten Programmcode zur Durchführung eines Verfahrens nach einem der vorhergehenden Ansprüche, wenn das Programm auf einen Rechner läuft.
